# FASCICULE DE BREVET EUROPEEN

(11) **EP 0 624 599 B1**
(45) Date de publication et mention de la délivrance du brevet: **30.07.1997**
(21) Numéro de dépôt: 94401069.3
(22) Date de dépôt: 11.05.1994
(51) Int. Cl.: C08B 37/16, C07C 271/22, A61K 7/48

(54) **Dérivés de mono (6-amino 6-désoxy)cyclodextrine substituée en position 6 par un reste d'alpha-aminoacide, leur procédé de préparation et leurs utilisations**
Derivate aus Mono (6-amino-6-desoxy)Cyclodextrin substituiert in Stellung 6 mit einem Alpha-Aminosäure-Rest Verfahren zu ihrer Herstellung und deren Verwendungen
Derivatives of mono (6-amino-desoxy)cyclodextrin substituted in position 6 with an alpha aminoacid group, process of their preparation and applications

(30) Priorité: 13.05.1993 FR 9305782
(43) Date de publication de la demande: 17.11.1994
(73) Titulaire: L'OREAL, 75008 Paris (FR)
(72) Inventeur: Galons, Hervé, F-75015 Paris (FR); Gnaim, Jallal, NL-2624 LZ Delft (NL); Maignan, Jean, F-93290 Tremblay les Gonesses (FR)
(74) Mandataire: Stalla-Bourdillon, Bernard

(56) Documents cités:
- WO-A-91/13100
- TETRAHEDRON LETTERS, vol.33, no.2, 7 Janvier 1992 pages 209 - 212 HELENE PARROT-LOPEZ ET AL. 'Vectorised Transport of Drugs : Synthesis of a new Glycosyl Derivative of Beta-Cyclodextrin'
- PATENT ABSTRACTS OF JAPAN vol. 13, no. 241 (C-604) 6 Juin 1989 & JP-A-01 051 402 (TOSOH CORP) 27 Février 1989
- JOURNAL OF CHEMICAL SOCIETY, CHEMICAL COMMUNICATIONS, 1975 pages 160 - 161 IAN J. CLIMIE ET AL. 'Labelling of Amino-acid Side-chains with 13C-labelled Electrophiles; Potential Application to the Probing of Active Sites of Enzymes.'
- TETRAHEDRON, vol.38, no.5, 1982 pages 697 - 711 IAN J. G. CLIMIE ET AL. 'C-Nuclear Magnetic Resonance Spectroscopy as a probe of Enzyme Environment'
- JOURNAL OF CHROMATOGRAPHY, vol.124, 1976, AMSTERDAM pages 92 - 96 MASAMI MAKITA ET AL. 'Gas-liquid chromatography of the N-isobutyloxycarbonyl methyl esters of non-protein amino acids.'

## Description

La présente invention a pour objet de nouveaux dérivés de mono(6-amino 6-désoxy) cyclodextrine substituée en position 6 par un reste d'α-aminoacide, leur procédé de préparation et leurs utilisations dans différents domaines de l'industrie en particulier en cosmétique, pharmacie et dermatologie, ces nouveaux dérivés étant susceptibles de former des complexes d'inclusion.

Ces nouveaux dénvés de cyclodextrine peuvent également trouver des applications dans d'autres domaines notamment en chimie analytique, dans l'industrie agro-alimentaire et également comme agent séquestrant de certains métaux de transition.

L'utilisation de molécules véhiculaires susceptibles d'accroître la solubilité en milieu aqueux de substances lipophiles ou de stabiliser des substances réputées instables en milieu aqueux ou encore d'obtenir une libération ciblée en fonction du temps ou du milieu environnant d'un principe actif est tout particulièrement recherchée dans les domaines cosmétique, pharmaceutique et dermatologique.

Il a ainsi été proposé dans la demande de brevet PCT WO 91/13100, l'utilisation de cyclodextrines substituées par divers groupements, notamment par des fonctions acides carboxyliques.

Cette demande de brevet décrit notamment la préparation et l'utilisation de dérivés de cyclodextrine résultant de la réaction de la mono(6-amino 6-désoxy)-β-cyclodextrine avec un anhydride dicarboxylique tel que l'anhydride succinique ou l'anhydride glutarique.

Elle décrit également, en son exemple 74, la N-((S)-4'-amino-4'-carboxy-butan-1-oyl)-6-amino-6-deoxy-β-cyclodextrine.

Les dérivés obtenus, porteurs d'une fonction acide carboxylique libre, présentent une meilleure solubilité mais celle-ci reste néanmoins insuffisante pour certaines applications et nécessitent de plus une neutralisation préalable, au moins partielle, des fonctions acides carboxyliques.

Il a également été décrit dans JP-A-105402, la préparation de β-cyclodextrines modifiées obtenues par réaction de β-aminocyclodextrine et d'un α-aminoacide N-amino protégé. Ces composés sont décrits comme trouvant une utilité en chromatographie liquide.

Après diverses recherches sur les cyclodextrines, on a constaté de façon tout à fait inattendue et surprenante qu'une nouvelle classe de dérivés de cyclodextrine permettait de remédier aux inconvénients rencontrés jusqu'à présent, notamment à ceux des dérivés de cyclodextrine de la demande PCT WO 91/13100.

Les nouveaux dérivés de cyclodextrine selon l'invention présentent non seulement l'avantage d'avoir une solubilité nettement supérieure aux dérivés précédemment connus et utilisés, mais également de pouvoir être employés directement, c'est-à-dire sans neutralisation préalable, tout en étant inoffensifs et bien tolérés par les muqueuses et la peau.

Ces principaux avantages sont dus essentiellement à la présence sur la molécule de cyclodextrine d'un reste d'α-aminoacide de telle sorte qu'en milieu aqueux, les dérivés de cyclodextrine selon l'invention se présentent sous forme bipolaires ou zwittérioniques.

La présente invention a donc pour objet à titre de produits industriels nouveaux des dérivés de mono(6-amino 6-désoxy) cyclodextrine substituée en position 6 par un reste d'α-aminoacide, ces dérivés répondant à la formule générale suivante : 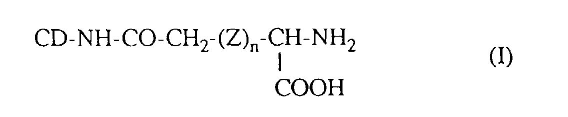 dans laquelle :
CD représente l'α,β ou γ cyclodextrine
n est 0 ou 1, et
Z représente un radical divalent choisi parmi :

   (i) -CH₂-

   (ii) -O-CH₂-

   (iii) -S-CH₂-

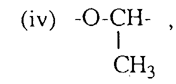

   et

   (v) -O-C₆H₄-CH₂-.
à l'exclusion des composés de formule (I) dans laquelle CD représente la β-cyclodextrine, n = 1 et Z = -CH₂-.

Selon un mode de réalisation préféré de l'invention, le radical CD est la β-cyclodextrine.

Parmi les dérivés de cyclodextrine de formule générale (I), on peut notamment citer:
- la mono[N-(amino-3 carboxy-3 propionyl)amino]-6 désoxy β-cyclodextrine, et
- la mono[N-(amino-2' carboxy-2' éthyl-4-phényloxyméthylcarbonyl-1)amino]-6 désoxy β-cyclodextrine.

La présente invention a également pour objet le procédé de préparation des dérivés de cyclodextrine de formule générale (I), ce procédé pouvant être représenté par le schéma réactionnel suivant : 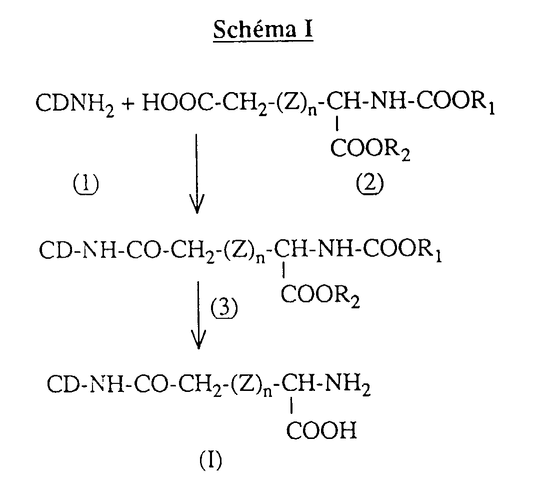
CD, Z et n ont les mêmes significations que celles données pour la formule (I),
R₁ représente le radical tertiobutyle ou le radical benzyle, et
R₂ représente le radical méthyle ou le radical benzyle.

Ce procédé consiste à faire réagir un α-aminoacide dicarboxylique (2) sous les formes L,D ou racémique (dont la fonction α-aminoacide a été protégée) avec une (6-amino 6-désoxy) cyclodextrine (1) en solution dans un solvant organique tel que le diméthylformamide en présence d'un excès de 1-hydroxybenzotriazole ne dépassant pas 20 % molaire par rapport à l'α-aminoacide dicarboxylique protégé (2) et d'un excès de dicyclohexylcarbodiimide ne dépassant pas 30 % molaire par rapport à l'α-aminoacide dicarboxylique protégé (2). La réaction est généralement réalisée sous agitation jusqu'à la disparition complète des produits de départ puis le mélange est filtré en vue d'éliminer la dicyclohexylurée formée. Après concentration sous vide l'amide obtenu (3) est recristallisé de préférence dans l'eau.

Selon un mode de réalisation préféré, le rapport molaire entre les composés (1) et (2) est généralement compris entre 1 et 1,5.

On procède ensuite à une déprotection des fonctions protégées du composé (3) dans des conditions opératoires qui sont fonction de la nature des groupes protecteurs utilisés.

Ainsi, les groupements benzyloxycarbonyles et benzyles sont déplacés par hydrogénation en présence de Pd/C (5 à 10 %) et les groupements tertiobutyloxycarbonyles sont clivés en présence d'acide trifluoroacétique, les fonctions esters méthyliques étant éliminées par saponification en présence de soude 1N.

La (6-amino 6-désoxy)-β-cyclodextrine (1) est un produit connu, préparé selon des méthodes connues par exemple celle décrite dans le brevet US 5.068.227. Certains des composés de formule (2) sont également disponibles dans le commerce auprès de la Société Bachem : il s'agit en particulier du N-benzyloxycarbonyl aspartate de benzyle (n = 0 et R₁ et R₂ = -CH₂C₆H₅) et du N-benzyloxycarbonyl glutamate de benzyle (Z = -CH₂- et R₁ et R₂ = -CH₂C₆H₅).

Les autres composés de formule (2) et leurs précurseurs dans laquelle Z est différent de -CH₂- sont nouveaux et peuvent être représentés par la formule générale suivante : 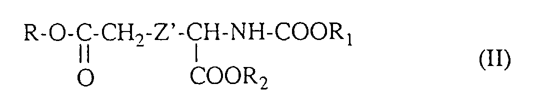 dans laquelle :
R représente un atome d'hydrogène ou le radical benzyle,
R₁ représente le radical tertiobutyle ou le radical benzyle,
R₂ représente le radical méthyle ou le radical benzyle, et
Z' représente un radical divalent choisi parmi :

   (i) -O-CH₂-

   (ii) -S-CH₂-

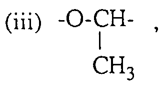

   et

   (iv) -O-C₆H₄-CH₂-.

Les composés intermédiaires de formule (II) sont obtenus selon le schéma réactionnel suivant :

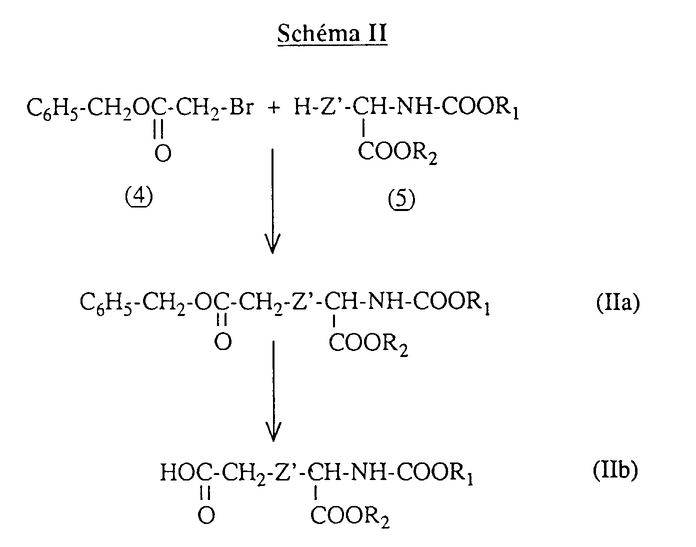

Le groupement H-Z'- représente le radical HO-CH₂-,HS-CH₂-, 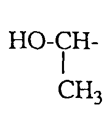 ou p-HO-C₆H₄-CH₂-, et
R₁ et R₂ ont les mêmes significations que celles données ci-dessus pour la formule (II).

La synthèse de ces composés consiste à faire réagir en milieu solvant organique, de préférence dans le diméthylformamide, en présence d'hydrure de lithium, du bromoacétate de benzyle (4) sur un ω-hydroxy- ou ω-mercapto-α-aminoacide protégé de formule (5), ce qui permet d'obtenir avec un bon rendement, le dérivé de O ou de S-alkylation de formule (IIa).

L'acide correspondant (IIb) est obtenu par hydrogénation en solution méthanol-chlorure de méthylène en présence de Pd/C 5 %.

Les ω-hydroxy-α-aminoacides protégés de formule (5) sont pour certains connus, en particulier le N-tertiobutyloxycarbonyl tyrosinate de méthyle (H-Z'= p-HO-C₆H₄-CH₂-, R₁ = -C(CH₃)₃ et R₂ = -CH₃) qui est commercialisé par la Société Bachem.

Les autres composés de formule (5) peuvent être facilement obtenus selon les méthodes conventionnelles de protection des fonctions α-aminoacides à partir d'α-aminoacides tels que la sérine, la thréonine et la cystéine.

Parmi les composés intermédiaires nouveaux de formule (II), on peut notamment citer le N-tertio-butyloxycarbonyl O-(benzyloxycarbonylméthyl)tyrosinate de méthyle et le N-tertio-butyloxycarbonyl O-(carboxyméthyl)tyrosinate de méthyle.

Les dérivés de cyclodextrine selon l'invention trouvent une application dans différents domaines de l'industrie.

Ces dérivés de cyclodextrine peuvent notamment être utilisés tels quels, c'est-à-dire sans inclusion d'actif dans des compositions notamment cosmétiques ou dermatologiques dans le traitement de la peau ou des cheveux, les dérivés de cyclodextrine étant susceptibles d'avoir un effet plastifiant.

Selon cette forme de réalisation, les dérivés de cyclodextrine selon l'invention sont généralement présents à une concentration comprise entre 0,1 et 10 % en poids par rapport au poids total de la composition.

Toutefois, l'utilisation de dérivés de cyclodextrine selon l'invention est plus particulièrement orientée vers la formation de complexes d'inclusion de diverses substances actives. Celles-ci sont de préférence des substances de type lipophile, ou bien encore des produits insolubles ou instables en milieu aqueux et sont choisis plus particulièrement parmi les principes actifs utilisés dans les domaines cosmétique, dermatologique et pharmaceutique.

Comme mentionné ci-dessus, la faculté des dérivés de cyclodextrine selon l'invention de pouvoir se présenter sous forme zwittérionique permet d'accroître la solubilité de ces principes actifs, ce qui, jusqu'à présent, n'avait pu être obtenu de façon satisfaisante.

Parmi les substances actives pouvant former des complexes d'inclusion ou pouvant être stabilisées, on peut notamment mentionner :
- les antioxydants et les antiradicaux libres tels que le butylhydroxytoluène (B.H.T.), le butylhydroxyanisole (B.H.A.), la vitamine E, les dérivés du para-tertiobutylbenzylidène camphre tels que ceux décrits dans le brevet français n° 2.624.508 et en particulier le 3',5'-ditertiobutyl 4'-hydroxy 3-benzylidène camphre,
- les agents anti-acnéiques, anti-vieillissement ou anti-photovieillissement comme l'acide rétinoique et ses isomères ainsi que certains dérivés de ceux-ci tels que ceux décrits dans la demande EP 465.343, dans les demandes WO 91/00793, dans la demande française n° 91 05394 ou encore dans la demande EP 514.264, ou comme le rétinol et ses esters notamment l'acétate ou le palmitate,
- les agents pour lutter contre le psoriasis tels que l'anthraline, les psoralènes, les rétinoïdes aromatiques comme le tigason et ses dérivés ou comme l'acide 6-[3-(1-adamantyl)-4-méthoxyphényl]-2-naphtoïque,
- les agents conservateurs et bactéricides, comme les isothiazolones substituées,
- les agents favorisant la repousse des cheveux ou évitant leur chute tels que le Minoxidil et ses dérivés ou les produits décrits dans les demandes de brevet français 2.606.635, 2.607.505, 2.618.068, 2.654.101, 2.663.327, et les demandes EP 304.649, EP 304.665, EP 353.323, EP 303.871, EP 356.271, EP 464.034, EP 177.581, EP 420.507, EP 408.442, et EP 459.890,
- les filtres solaires,
- les agents d'hydratation et/ou de plastification du stratum cornéum tels que les α-hydroxyacides, les dérivés de thiamorpholinone tels que ceux décrits dans les brevets français n° 2.525.106 et 2.525.220, l'acide pyrrolidone carboxylique et les sérines, les dérivés salicylés tels que ceux décrits dans la demande EP 378.936 et notamment l'acide n-octanoyl-5-salicylique,
- les agents de reconstitution de la barrière lipidique tels que les céramides et leurs dérivés, en particulier ceux décrits dans le brevet français n° 2.588.256 ainsi que dans les demandes EP 420.722 et EP 500.437,
- les colorants capillaires difficilement solubles en milieu aqueux tels que les colorants anthraquinoniques, les colorants azoïques et les dérivés nitrés de la série benzénique, ou encore les colorants pouvant être stabilisés tels que les indoles et les indolines substitués,
- les agents dépigmentants tels que l'hydroquinone, l'acide kojique, l'arbutine et ses dérivés,
- les agents anti-inflammatoires tels que les inhibiteurs de lipoxygénase et/ou de cyclooxygénase,
- des stéroïdes comme l'hydrocortisone et l'hydroxy-17 corticostérone,
- des agents anti-viraux et anticancéreux comme le 5-fluorouracile.

Les compositions cosmétiques, pharmaceutiques ou dermatologiques contenant de tels complexes d'inclusion peuvent se présenter sous diverses formes, notamment sous forme de lotions aqueuses ou hydroalcooliques, de gels ou de dispersions et sont présents en une proportion comprise entre 0,1 et 30 % par rapport au poids total des compositions.

Les dérivés de cyclodextrine selon l'invention sont également susceptibles de former des complexes d'inclusion avec certains sels métalliques, notamment les sels de métaux intervenant dans le processus d'oxydo-réduction lors de la coloration ou décoloration des cheveux ou lors de la coloration ou de la lutte contre le vieillissement de la peau.

Les dérivés de cyclodextrine selon l'invention trouvent en outre certaines applications dans la formation de complexes avec des molécules d'aminoacides par l'intermédiaire d'un métal de transition permettant ainsi de séquestrer ces métaux, les dérivés de cyclodextrine selon l'invention étant alors susceptibles d'agir en tant qu'agents protecteurs ou de détoxification de ces métaux, notamment dans des produits cosmétiques.

Bien d'autres utilisations peuvent être envisagées pour les dérivés de cyclodextrine selon l'invention notamment en électrophorèse capillaire, comme phase chirale-chromatographique, ou encore pour la formation de complexes dans le domaine agro-alimentaire.

On va maintenant donner à titre d'illustration plusieurs exemples de préparation des dérivés de cyclodextrine selon l'invention et d'utilisation de ces dérivés.

### EXEMPLES DE PREPARATION

La révélation des chromatographies sur couche mince de silice (CCM) a été effectuée par UV ou après pulvérisation de H₂SO₄ 10 % puis chauffage ou par la ninhydrine en solution dans l'éthanol puis chauffage. Les spectres de RMN ont été enregistrés sur un appareil Brucker 270 (Référence interne TMS).

### EXEMPLE 1 : Préparation de la mono[N-(amino-4 carboxy-4 butyryl)amino]-6 désoxy β-cyclodextrine

(a) *Mono [N-(benzyloxycarbonylamino-4 benzyloxycarbonyl-4 butyryl)amino]-6 désoxy β-cyclodextrine*
   A une solution de 0,635 g (1,6.10⁻³ mole) de N-benzyloxycarbonyl α-glutamate de benzyle dans 20 ml de diméthylformamide (DMF), on ajoute 0,209 g de 1-hydroxybenzotriazole et 0,385 g (1.855.10⁻³ mole) de dicyclohexylcarbodiimide. Après 30 minutes d'agitation à 20°C, on ajoute 1,5 g (1,325.10⁻³ mole) de mono(6-amino-6-désoxy)-β-cyclodextrine et le mélange est agité 24 heures à température ambiante. Après filtration de la dicyclohexylurée, la solution organique est concentrée sous vide. Le solide obtenu est recristallisé dans l'eau et se présente après séchage sous forme d'un solide incolore ayant un point de fusion supérieur à 240°C (Rdt : 68-78%).
   CCM : Rf = 0,38 (Eluant : Acétate d'éthyle : 36 % ; isopropanol 36 % ; ammoniaque concentrée : 21 % ; eau 7 %)
   RMN (DMSOd₆) : CH₂CH₂CO = 1,83-1,93 ; CH₂CH₂CO = 2,25 ; CH-CH₂ = 4,10 ; anomériques = 4,82 ; CH₂C₆H₅ = 5,01; CH₂C₆H₅ = 5,10; C₆H₅ = 7,31-7,34.
(b) *Mono[N-(amino-4 carboxy-4 butyryl)amino]-6 désoxy β-cyclodextrine*
   A une solution de 1,5 g (1.10⁻³ mole) du composé préparé ci-dessus en (a) dans un mélange de 20 ml d'eau et de 50 ml de méthanol, on ajoute 200 mg de Pd/C (10 %) puis agite vigoureusement à 20°C sous hydrogène pendant 6 heures. Après élimination de l'hydrogène, le mélange est filtré, le catalyseur est rincé par 3 fois 5 ml d'eau et le filtrat est concentré sous vide. On obtient un solide incolore ayant un point de fusion supérieur à 240°C avec un rendement de 86%.
   CCM : Rf = 0,20 (Eluant : identique à 1(a) ci-dessus)
   RMN(DMSOd₆) : CH₂CH₂CO = 1,90; CH₂CH₂CO = 2,29 ; anomériques = 4,82-4,85 ; β-CD = 3,29-3,35 et 3,56-3,65.
   Solubilité dans l'eau supérieure à 1000 g/l.

### EXEMPLE 2 : Préparation de la mono[N-(amino-3 carboxy-3 propionyl)amino]-6 désoxy β-cyclodextrine

(a) *Mono [N-(benzyloxycarbonylamino-3 benzyloxycarbonyl-3 propionyl)amino]-6 désoxy β-cyclodextrine*
   A une solution de 0,611 g (1,6.10⁻³ mole) de N-benzyloxycarbonyl-aspartate de benzyle dans 20 ml de diméthylformamide, on ajoute 0,209g de 1-hydroxybenzotriazole et 0,385 g (1,855.10⁻³ mole) de dicyclohexylcarbodiimide. Après 30 minutes d'agitation à 20°C, on ajoute 1,5 g (1,325.10⁻³ mole) de mono(6-amino-6-désoxy)-β-cyclodextrine, puis on agite le mélange pendant 24 heures à température ambiante.
   On filtre la dicyclohexylurée formée puis la solution organique est concentrée sous vide. Le solide obtenu est recristallisé dans l'eau.
   On obtient un solide incolore, de point de fusion supérieur à 240°C, avec un rendement compris entre 71 et 86 %.
   CCM : Rf = 0,62 (Eluant : acétate d'éthyle : 36 % ; isopropanol : 36 %, ammoniaque concentrée : 21 % ; eau : 7 %)
   RMN : (DMSO d₆) = CH₂CO : 3,05 et 3,15 ; β-CD : 3,30 et 3,68 ; anomériques : 4,82 ; hydroxyles : 5,61-5,74 ; C₆H₅ : 7,33-7,37.
(b) *Mono[N-(amino-3 carboxy-3 propionyl)amino]-6 désoxy β-cyclodextrine*
   A une solution de 1,48 g (1.10⁻³ mole) du composé obtenu ci-dessus en (a) dans un mélange de 20 ml d'eau et de 50 ml de méthanol, on ajoute 200 mg de Pd/C (10 %), puis agite vigoureusement, à 20°C, sous hydrogène pendant 6 heures.
   Après élimination de l'hydrogène, le mélange est filtré, le catalyseur est rincé 3 fois par 5 ml d'eau, puis le filtrat est concentré sous vide.
   On obtient un solide incolore, de point de fusion supérieur à 240°C, avec un rendement de 91 %.
   Solubilité dans l'eau supérieure à 1000 g/l.
   CCM : Rf = 0,375 (Eluant identique à celui utilisé en 2 (a) ci-dessus)
   RMN : (DMSO d₆)= CH₂CO : 2,90 ; β-CD : 3,31 et 3,73 ; anomériques : 4,82 ; hydroxyles : 5,65-5,79.

### EXEMPLE 3 : Préparation de la mono[N-(amino-2'carboxy-2'éthyl-4-phényloxyméthylcarbonyl-1)amino]-6 désoxy β-cyclodextrine

*A) Mono [N-(tertiobutyloxycarbonylamino-2'méthoxycarbonyl-2' éthyl-4-phényloxyméthylcarbonyl-1)amino]-6* désoxy β-cyclodextrine
   (a) A une solution de 5,5 g (18,7.10⁻³ mole) de N-tertiobutyloxycarbonyltyrosinate de méthyle, on ajoute à température ambiante, 0,165 g (20,5.10⁻³ mole) d'hydrure de lithium. Après 15 minutes d'agitation, on ajoute 4,719 g (20,5.10⁻³ mole) de bromoacétate de benzyle. Le mélange est agité pendant 5 heures à 40°C, puis concentré sous vide et le produit d'O-alkylation est séparé par chromatographie sur silice par élution à l'aide d'un mélange d'acétate d'éthyle 50 % et d'éther de pétrole 50 %. On obtient une huile incolore, avec un rendement de 82 %.
      CCM : Rf = 0,92 (Eluant : acétate d'éthyle 100 %) ; Rf = 0,30 (éluant acétate d'éthyle 50 % ; cyclohexane 50 %).
      RMN (CDCl₃) : (CH₃)₃C = 1,42 ; CH₂-C₆H₄ = 3,05 ; OCH₃ = 3,7 ; CHCH₂ = 4,6 ; OCH₂CO = 4,64 ; NH = 4,95 ; CH₂C₆H₅ = 5,25 ; CH aro(aryloxy) = 6,83 et 7,03 ; aro(benzyle) = 7,27-7,37 ;
   (b) Le produit obtenu en (a) est repris par 50 ml d'un mélange de méthanol 50 % et de chlorure de méthylène 50 %, puis on ajoute à la solution 50 mg de Pd/C (5 %), et on agite sous atmosphère d'hydrogène.
      Lorsque l'absorption d'hydrogène a cessé (après environ 50 mn), le milieu est filtré, le catalyseur est rincé 2 fois par 10 ml de chlorure de méthylène, puis la solution est évaporée.
      On obtient une huile jaune avec un rendement de 96 %.
      CCM : Rf = 0,45 (éluant : acétate d'éthyle 100 %)
      Rf = 0,15 (éluant : acétate d'éthyle 50 %-cyclohexane 50 %).
      RMN (CDCl₃) : (CH₃)₃C = 1,45 ; CH₂-C₆H₄ = 3,01 ; OCH₃ = 3,72 ; CHCH₂ = 4,53; OCH₂CO = 4,64 ; NH = 4,95 ; CH aro(aryloxy) = 6,81 et 7,02.
   (c) L'acide ainsi obtenu est alors mis à réagir avec la (6-amino-6-désoxy)-β-cyclodextrine dans les mêmes conditions que celles de l'exemple 1(a).
      On obtient le produit attendu sous forme d'un solide incolore avec un rendement de 78 %.
      CCM : Rf = 0,30 (Eluant : acétate d'éthyle 36 % ; isopropanol 36 % ; ammoniaque concentrée 21 % ; eau 7 %)
      RMN(DMSO+D₂O) : C(CH₃)₃ = 1,43 ; β-C+OCH₃ = 3,2-3,4 et 3,5-3,6 ; CHCH₂ = 4,50 ; OCH₂CO = 4,6 ; CH aro(aryloxy) = 6,83 et 7,08.
*B) Mono[N-(amino-2'carboxy-2'éthyl-4-phényloxyméthylcarbonyl-1)amino]-6-désoxy β-cyclodextrine*
   Le composé obtenu précédemment en (A)(c) est dissout dans 10 ml d'une solution 1:1 d'acide trifluoroacétique dans le chlorure de méthylène. Après 2 heures à température ambiante, on concentre le mélange, le reprend dans 10 ml d'eau, puis ajoute 30 ml d'une solution d'hydroxyde de sodium 1N. Après 3 heures d'agitation à température ambiante, le milieu est concentré sous vide et l'on reprend le solide par de l'eau. Après purification sur une colonne de Sephadex, le produit est lyophilisé.
   On obtient une poudre incolore, de point de fusion supérieur à 240°C, avec un rendement de 76 %.
   RMN (DMSO) : β-CD : 3,2-3,4 et 3,5-3,6 ; anomériques = 4,85 ; aromatiques = 6,87 et 7,12.
   CMM : Rf = 0,23 (Eluant : acétate d'éthyle : 36 % ; isopropanol 36 % ; ammoniaque concentrée 21 %, eau 7 %).
   Solubilité dans l'eau : 900 g/l.

### EXEMPLES D'UTILISATION

### EXEMPLE A :Complexe d'inclusion du minoxidil dans la mono[N-(amino-3 carboxy-3 propionyl)amino]-6 désoxy β-cyclodextrine

A une solution de 114 mg du composé préparé à l'exemple 2(b) dans 0,3 ml d'eau, on ajoute à 70°C, 22 mg de minoxidil. Après 15 minutes d'agitation à 70°C, le mélange est refroidi à la température ambiante, puis filtré. La solution limpide obtenue est lyophilisée.

D'après les dosages en HPLC et en RMN, ce complexe contient 15,4 % de minoxidil, ce qui correspond à un rapport de 1,1 molécule de minoxidil pour 1 molécule du composé de l'exemple 2(b).

L'inclusion du minoxidil dans le dérivé de cyclodextrine selon l'exemple 2(b) accroit sa solubilité dans l'eau d'un facteur de l'ordre de 40, celle-ci étant alors de 110 g/l.

### EXEMPLE B :Complexe d'inclusion du minoxidil dans la mono[N-(amino-4 carboxy-4 butyryl)amino]-6 désoxy β-cyclodextrine

Selon le même mode opératoire que décrit à l'exemple A, on prépare un complexe d'inclusion du minoxidil dans la mono[N-(amino-4 carboxy-4 butyryl)amino-6 désoxy] β-cyclodextrine à partir de 128 mg du composé préparé à l'exemple 1(b) et de 21 mg de minoxidil.

D'après le dosage en RMN, ce complexe contient 1 molécule de minoxidil pour 1 molécule du composé de l'exemple 1(b).

L'inclusion du minoxidil dans le dérivé de cyclodextrine selon l'exemple 1(b) accroît sa solubilité dans l'eau d'un facteur de l'ordre de 40, celle-ci étant alors de 110 g/l.

### EXEMPLE C :Complexe d'inclusion du 5-fluorouracile dans la mono[N-(amino-4carboxy-4 butyryl)amino]-6 désoxy β-cyclodextrine

Selon le même opératoire que décrit à l'exemple A, on prépare un complexe d'inclusion du 5-fluorouracile dans la mono[N-(amino-4 carboxy-4 butyryl)amino-6 désoxy] β-cyclodextrine à partir de 128 mg du composé préparé à l'exemple 1(b) et de 13 mg de 5-fluorouracile.

D'après le dosage en RMN, ce complexe contient 1 molécule de 5-fluorouracile pour 1 molécule du composé de l'exemple 1(b).

L'inclusion du 5-fluorouracile dans le dérivé de cyclodextrine selon l'exemple 1(b), accroît sa solubilité dans l'eau d'un facteur de l'ordre de 100, celle-ci étant alors de 80 g/l.

### EXEMPLE D :Complexe d'inclusion de l'acide 6-[3-(1-adamantyl)-4-méthoxy phényl]-2-naphtoïque dans la mono[N-(amino-4 carboxy-4 butyryl)amino]-6 désoxy β-cyclodextrine

Selon le même mode opératoire que décrit à l'exemple A, on prépare un complexe d'inclusion de l'acide 6-[3-(1-adamantyl)-4-méthoxy phényl]-2-naphtoïque dans la mono[N-(amino-4 carboxy-4 butyryl)amino-6 désoxy] β-cyclodextrine à partir de 128 mg du composé préparé à l'exemple 1(b) et de 10 mg d'acide 6-[3-(1-adamantyl)-4-méthoxy phényl]-2-naphtoïque.

D'après le dosage en RMN, ce complexe contient 1 molécule d'acide 6-[3-(1-adamantyl)-4-méthoxy phényl]-2-naphtoïque pour 8 molécules du composé de l'exemple 1(b).

L'inclusion de l'acide 6-[3-(1-adamantyl)-4-méthoxy phényl]-2-naphtoïque dans le dérivé de cyclodextrine selon l'exemple 1(b) accroît sa solubilité dans l'eau d'un facteur de l'ordre de 1.000, celle-ci étant alors de 4 g/l.

### EXEMPLE E :Complexe d'inclusion de l'hydroxy-17 corticostérone dans la mono[N-(amino-4 carboxy-4 butyryl)amino]-6 désoxy β-cyclodextrine

Selon le même mode opératoire que décrit à l'exemple A, on prépare un complexe d'inclusion de l'hydroxy-17 corticostérone dans la mono-(6-γ-glutamyl-amino-6 désoxy)-β-cyclodextrine à partir de 128 mg du composé préparé à l'exemple 1(b) et de 20 mg d'hydroxy-17 corticostérone.

D'après le dosage en RMN, ce complexe contient 1 molécule d'hydroxy-17 corticostérone pour 2 molécules du composé de l'exemple 1(b).

L'inclusion de l'hydroxy-17 corticostérone dans le dérivé de cyclodextrine selon l'exemple 1(b) accroît sa solubilité dans l'eau d'un facteur de l'ordre de 200, celle-ci étant alors de 11 g/l.

## Revendications

1. Dérivés de mono(6-amino 6-désoxy) cyclodextrine substituée en position 6 par un reste d'α-aminoacide, caractérisés par le fait qu'ils répondent à la formule générale suivante : 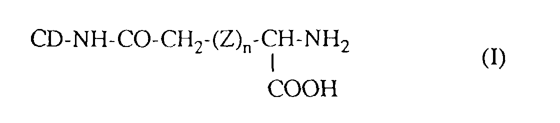 dans laquelle :
CD représente l'α, β ou γ cyclodextrine
n est 0 ou 1, et
Z représente un radical divalent choisi parmi :
(i) -CH₂-
(ii) -O-CH₂-
(iii) -S-CH₂-, 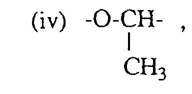
et
(v) -O-C₆H₄-CH₂-.
à l'exclusion des composés de formule (I) dans laquelle CD représente la β-cyclodextrine, n = 1 et Z = -CH₂-.

2. Dérivés de cyclodextrine selon la revendication 1, caractérisés par le fait que la cyclodextrine est la β-cyclodextrine.

3. Dérivés de cyclodextrine selon l'une des revendications 1 et 2, caractérisés par le fait qu'ils sont choisis parmi :
- la mono[N-(amino-3 carboxy-3 propionyl)amino]-6 désoxy β-cyclodextrine,et
- la mono[N-(amino-2' carboxy-2' éthyl-4-phényloxyméthylcarbonyl-1)amino]-6 désoxy β-cyclodextrine.

4. Procédé de préparation des dérivés de cyclodextrine selon les revendications 1 à 3, caractérisé par le fait que l'on fait réagir en solution dans un solvant organique un α-aminoacide dicarboxylique dont la fonction α-aminoacide a été protégée et ayant la formule suivante : 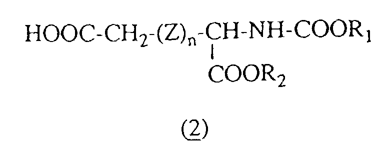 dans laquelle :
Z et n sont tels que définis à la revendication 1,
R₁ représente le radical tertiobutyle ou le radical benzyle, et
R₂ représente le radical méthyle ou le radical benzyle,
avec une mono(6-amino 6-désoxy) cyclodextrine de formule : CDNH₂
dans laquelle : CD est tel que défini à la revendication 1, la réaction étant effectuée en présence de 1-hydroxybenzotriazole et de dicyclohexylcarbodiimide, et que l'on procède ensuite à la déprotection de la fonction α-amino acide protégée.

5. Procédé selon la revendication 4, caractérisé par le fait que le solvant organique est le diméthylformamide.

6. Procédé selon la revendication 4 ou 5, caractérisé par le fait que le 1-hydroxybenzotriazole est utilisé en un excès molaire ne dépassant pas 20 % par rapport à l'α-aminoacide dicarboxylique protégé et le dicyclohexylcarbodiimide en un excès molaire ne dépassant pas 30 % par rapport à l'α-aminoacide dicarboxylique protégé.

7. Procédé selon l'une quelconque des revendications 4 à 6, caractérisé par le fait que le rapport molaire entre la mono(6-amino 6-désoxy) cyclodextrine et l'α-amino acide dicarboxylique protégé est compris entre 1 et 1,5.

8. Procédé selon l'une quelconque des revendications 4 à 7, caractérisé par le fait que la mono(6-amino 6-désoxy) cyclodextrine est la mono(6-amino 6-désoxy)-β-cyclodextrine.

9. Procédé selon l'une quelconque des revendications 4 à 8, caractérisé par le fait que la déprotection est effectuée par hydrogénation en présence de Pd/C (5 à 10 %) lorsque dans la formule (2), R₁ et R₂ représentent un radical benzyle.

10. Procédé selon l'une quelconque des revendications 4 à 8, caractérisé par le fait que la déprotection est effectuée en présence d'acide trifluoroacétique lorsque dans la formule (2), R₁ représente un radical tertiobutyle, la déprotection de la fonction ester étant obtenue par saponification en présence de soude.

11. Procédé selon l'une quelconque des revendications 4 à 9, caractérisé par le fait que l'α-amino acide dicarboxylique protégé est choisi parmi :
- le N-benzyloxycarbonyl aspartate de benzyle,
- le N-benzyloxycarbonyl glutamate de benzyle, et
- le N-tertio-butyloxycarbonyl O-carboxyméthyl tyrosinate de méthyle.

12. Produits intermédiaires dans la synthèse des dérivés de cyclodextrine caractérisés par le fait qu'ils répondent à la formule générale suivante : 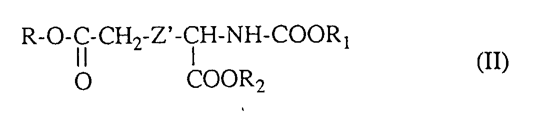 dans laquelle :
R représente un atome d'hydrogène ou le radical benzyle,
R₁ représente le radical tertiobutyle ou le radical benzyle,
R₂ représente le radical méthyle ou le radical benzyle, et
Z' représente un radical divalent choisi parmi :
(i) -O-CH₂-
(ii) -S-CH₂- 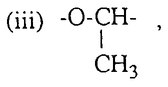
et
(iv) -O-C₆H₄-CH₂- .

13. Produits intermédiaires selon la revendication 12, caractérisés par le fait qu'ils sont choisis parmi : le N-tertio-butyloxycarbonyl O-(benzyloxycarbonylméthyl)tyrosinate de méthyle et le N-tertio-butyloxycarbonyl O-(carboxyméthyl)tyrosinate de méthyle.

14. Procédé de préparation des produits intermédiaires selon les revendications 12 et 13, caractérisé par le fait qu'il consiste à faire réagir dans un solvant organique, en présence d'hydrure de lithium, du bromoacétate de benzyle sur un ω-hydroxy- ou ω-mercapto-α-amino acide protégé de formule : 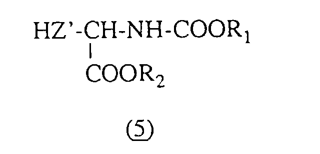 dans laquelle :
le groupement HZ'- représente le radical HO-CH₂, HS-CH₂-, 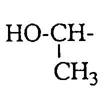 ou p-OH-C₆H₄-CH₂-,
R₁ représente le radical tertiobutyle ou le radical benzyle, et
R₂ représente le radical méthyle ou le radical benzyle, et à soumettre éventuellement le produit obtenu de formule : 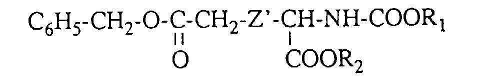 à une déprotection par hydrogénation en solution méthanol/chlorure de méthylène en présence de Pd/C en vue d'obtenir l'acide correspondant.

15. Procédé selon la revendication 14, caractérisé par le fait que le ω-hydroxy α-amino acide protégé de formule (5) est le N-tertiobutyloxycarbonyl tyrosinate de méthyle.

16. Composition cosmétique ou dermatologique, caractérisée par le fait qu'elle contient de 0,1 à 10 % en poids d'au moins un dérivé de cyclodextrine selon les revendications 1 à 3 ou obtenu selon les revendications 4 à 11.

17. Utilisation des dérivés de cyclodextrine selon les revendications 1 à 3, pour la formation de complexes d'inclusion de substances actives de nature lipophile ou insolubles ou instables en milieu aqueux.

18. Utilisation selon la revendication 17, caractérisée par le fait que les substances actives sont choisies parmi les antioxydants, les antiradicaux libres, les agents anti-acnéiques, les agents anti-vieillissement, les agents anti-psoriatiques, les agents conservateurs, les agents bactéricides, les agents favorisant la repousse des cheveux, les agents d'hydratation et/ou de plastification du stratum cornéum, les filtres solaires, les agents de reconstitution de la barrière lipidique, les colorants capillaires, les agents dépigmentants, les agents anti-inflammatoires, les agents anti-viraux, les agents anticancéreux et les stéroïdes.

19. Composition cosmétique ou dermatologique, caractérisée par le fait qu'elle contient un complexe d'inclusion formé à l'aide d'un dérivé de cyclodextrine selon l'une quelconque des revendications 1 à 3 et d'au moins une substance active telle que définie selon la revendication 18.

20. Composition cosmétique ou dermatologique selon la revendication 19, caractérisée par le fait qu'elle contient le complexe d'inclusion en une proportion comprise entre 0,1 et 30 % par rapport au poids total de la composition.

## Patentansprüche

1. Mono-(6-amino-6-desoxy)-cyclodextrinderivate mit einer Substitution durch einen α-Aminosäurerest in der Stellung 6, dadurch gekennzeichnet, daß sie der folgenden allgemeinen Formen entsprechen: 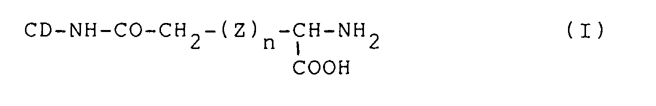 worin:
CD α-, β- oder γ-Cyclodextrin darstellt,
n die Zahl 0 oder 1 bedeutet, und
Z eine zweiwertige Gruppe darstellt, die unter den folgenden Gruppen ausgewählt ist:
(i) -CH₂-
(ii) -O-CH₂-
(iii) -S-CH₂- 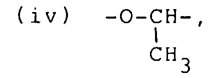
sowie
(v) -O-C₆-H₄-CH₂-
mit Ausnahme von Verbindungen gemäß der Formel (I), worin CD β-Cyclodextrin, n = 1 und Z = -CH₂- bedeuten.

2. Cyclodextrinderivate nach Anspruch 1, dadurch gekennnzeichnet, daß es sich beim Cyclodextrin um β-Cyclodextrin handelt.

3. Cyclodextrinderivate nach einem der Ansprüche 1 und 2, dadurch gekennzeichnet, daß sie unter den folgenden Verbindungen ausgewählt sind:
Mono-[N-(3-amino-3-carboxypropionyl)-amino]-6-desoxy-β-cyclodextrin, und
Mono-[(2'-amino-2'-carboxy-4-ethyl-1-phenyloxymethylcarbonyl)-amino]-6-desoxy-β-cyclodextrin.

4. Verfahren zur Herstellung von Cyclodextrinderivaten nach den Ansprüchen 1 bis 3, dadurch gekennzeichnet, daß eine α-Aminodicarbonsäure in Lösung in einem organischen Lösungsmittel, deren α-Aminosäurefunktion geschützt ist und die der folgenden Formel entspricht: 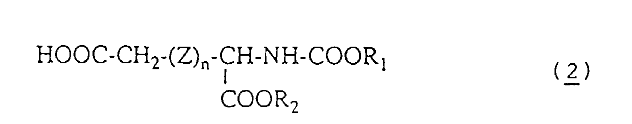 worin
Z und n die in Anspruch 1 angegebenen Bedeutungen aufweisen,
R₁ die tert-Butylgruppe oder die Benzylgruppe bedeutet, und
R₂ die Methyl- oder Benzylgruppe darstellt,
zusammen mit einem Mono-(6-amino-6-desoxy)-cyclodextrin der Formel CDNH₂ zur Reaktion gebracht wird,
worin:
CD die in Anspruch 1 angegebene Bedeutung aufweist, wobei
die Reaktion in Anwesenheit von 1-Hydroxybenzotriazol und Dicyclohexylcarbodiimid durchgeführt wird und daß anschließend die Synthese nach der Entfernung der Schutzgruppen an der α-Aminosäurefunktion fortgeführt wird.

5. Verfahren nach Anspruch 4, dadurch gekennzeichnet, daß das organische Lösungsmittel Dimethylformamid ist.

6. Verfahren nach Anspruch 4 oder 5, dadurch gekennzeichnet, daß das 1-Hydroxybenzotriazol in einem molaren Überschuß eingesetzt wird, der 20 % in bezug auf die geschützte α-Aminodicarbonsäure nicht übersteigt, wobei der molare Überschuß an Dicyclohexylcarbodiimid 30 % in bezug auf die geschützte α-Aminodicarbonsäure nicht übersteigt.

7. Verfahren nach einem der Ansprüche 4 bis 6, dadurch gekennzeichnet, daß das molare Verhältnis zwischen dem Mono-(6-amino-6-desoxy)-cyclodextrin und der geschützten α-Aminodicarbonsäure im Bereich zwischen 1 und 1,5 liegt.

8. Verfahren nach einem der Ansprüche 4 bis 7, dadurch gekennzeichnet, daß das Mono-(6-amino-6-desoxy)-cyclodextrin als Mono-(6-amino-6-desoxy)-β-cyclodextrin vorliegt.

9. Verfahren nach einem der Ansprüche 4 bis 8, dadurch gekennzeichnet, daß die Entfernung der Schutzgruppen durch Hydrierung in Anwesenheit von Pd/C (5 bis 10 %) unter der Voraussetzung durchgeführt wird, daß in der Formel (2) R₁ und R₂ eine Benzylgruppe darstellen.

10. Verfahren nach einem der Ansprüche 4 bis 8, dadurch gekennzeichnet, daß die Entfernung der Schutzgruppen in Anwesenheit von Trifluoressigsäure unter der Voraussetzung durchgeführt wird, daß in der Formel (2) R₁ eine tert-Butylgruppe bedeutet, wobei die Entfernung der Schutzgruppen an der Esterfunktion durch die Verseifung in Anwesenheit von Natriumcarbonat erfolgt.

11. Verfahren nach einem der Ansprüche 4 bis 9, dadurch gekennzeichnet, daß die geschützte α-Aminodicarbonsäure unter den folgenden Verbindungen ausgewählt wird:
Benzyl-N-benzyloxycarbonylaspartat
Benzyl-N-benzyloxycarbonylglutamat, und
Methyl-N-tert-butyloxycarbonyl-O-carboxymethyltyrosinat.

12. Zwischenprodukte für die Synthese von Cyclodextrinderivaten, dadurch gekennzeichnet, daß sie der folgenden allgemeinen Formel entsprechen: 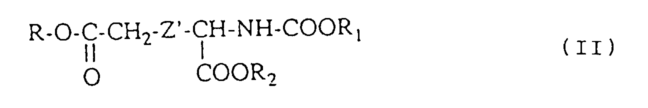 worin:
R ein Wasserstoffatom oder die Benzylgruppe,
R₁ die tert-Butylgruppe oder die Benzylgruppe,
R₂ die Methyl- oder Benzylgruppe, und
Z' eine zweiwertige Gruppe bedeuten, die aus den folgenden Gruppen ausgewählt sind:
(i) -O-CH₂-
(ii) -S-CH₂- 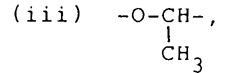
sowie
(iv) -O-C₆H₄-CH₂.

13. Zwischenprodukte nach Anspruch 12, dadurch gekennzeichnet, daß sie unter den folgenden Verbindungen ausgewählt sind:
Methyl-N-tert-butyloxycarbonyl-O-(benzyloxycarbonylmethyl)-tyrosinat, sowie
Methyl-N-tert-butyloxycarbonyl-O-(carboxymethyl)-tyrosinat.

14. Verfahren zur Herstellung von Zwischenprodukten nach den Ansprüchen 12 und 13, dadurch gekennzeichnet, daß es darin besteht, daß Benzylbromacetat mit einer geschützten ω-Hydroxy- oder ω-Mercapto-α-aminosäure der Formel 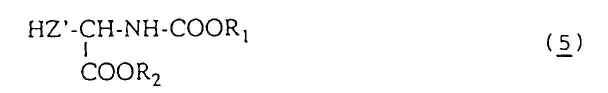 in einem organischen Lösungsmittel in Anwesenheit von Lithiumhydrid zur Reaktion gebracht wird, worin
die Gruppierung HZ'- die Gruppe -HO-CH₂, HS-CH₂-, 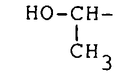 oder O-OH-C₆H₄-CH₂ bedeutet,
R₁ die tert-Butyl- oder Benzylgruppe, und
R₂ die Methyl- oder Benzylgruppe
bedeuten, wobei gegebenenfalls bei dem gewonnenen Produkt der Formel 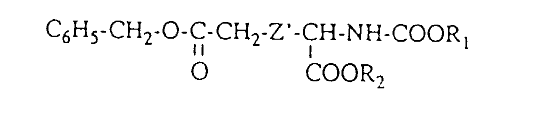
eine Entfernung der Schutzgruppen durch Hydrierung in einer Lösung von Ethanol/Methylenchlorid in Anwesenheit von Pd/C zur Gewinnung der entsprechenden Säure vorgenommen wird.

15. Verfahren nach Anspruch 14, dadurch gekennzeichnet, daß die geschützte ω-Hydroxy-α-aminosäure gemäß der Formel (5) als Methyl-N-tert-butyloxycarbonyltyrosinat vorliegt.

16. Kosmetische oder dermatologische Zubereitung, dadurch gekennzeichnet, daß sie 0,1 bis 10 Gew.-% mindestens eines Cyclodextrinderivats nach den Ansprüchen 1 bis 3 oder gemäß der Synthese nach den Ansprüchen 4 bis 11 enthält.

17. Verwendung von Cyclodextrinderivaten nach den Ansprüchen 1 bis 3 zur Bildung von Komplexen zum Einschluß von Wirkstoffen lipophiler Natur oder solcher, die unlöslich oder in wäßrigem Milieu instabil sind.

18. Verwendung nach Anspruch 17, dadurch gekennzeichnet, daß die Wirkstoffe unter Antioxidantien, Stoffen gegen freie Radikale, Mittel gegen Akne, Mittel gegen das Altern, Mittel gegen Psoriasis, Konservierungsmitteln, Bakteriziden, Haarwuchsmitteln, Hydratisierungsmitteln oder/und Mittel zum Elastischmachen der Hornhaut, Sonnenfiltersubstanzen, Mittel zum Wiederherstellen der Lipidbarriere, Haarfärbemitteln, depigmentierenden Mitteln, Entzündungshemmern, antiviralen Mitteln, Cancerostatika und Steroiden ausgewählt werden.

19. Kosmetische oder dermatologische Zubereitung, dadurch gekennzeichnet, daß sie einen mit Hilfe eines Dextrinderivats nach einem der Ansprüche 1 bis 3 gebildeten Einschlußkomplex und mindestens einen Wirkstoff der in anspruch 18 angegebenen Bedeutung enthält.

20. Kosmetische oder dermatologische Zubereitung nach Anspruch 19, dadurch gekennzeichnet, daß sie den Einschlußkomplex in einem Mengenverhältnis zwischen 0,1 und 30 Gew.-% in bezug auf das Gesamtgewicht der Zusammensetzung enthält.

## Claims

1. Mono(6-amino-6-deoxy)cyclodextrin derivatives substituted in the 6-position by an α-amino acid residue, characterized in that they correspond to the following general formula: 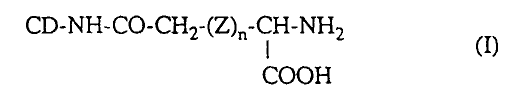 in which:
CD represents α-, β- or γ-cyclodextrin,
n is 0 or 1, and
Z represents a divalent radical chosen from:
(i) -CH₂-
(ii) -O-CH₂-
(iii) -S-CH₂-, 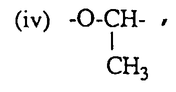
and
(v) -O-C₆H₄-CH₂-
with the exclusion of the compounds of formula (I) in which CD represents β-cyclodextrin, n = 1 and Z = -CH₂-.

2. Cyclodextrin derivatives according to Claim 1, characterized in that the cyclodextrin is β-cyclodextrin.

3. Cyclodextrin derivatives according to either of Claims 1 and 2, characterized in that they are chosen from:
- mono-6-[N-(3-amino-3-carboxypropionyl)-amino]deoxy-β-cyclodextrin, and
- mono-6-[N(4-(2'-amino-2'-carboxyethyl)-phenyloxymethyl-1-carbonyl)amino]deoxy-β-cyclodextrin.

4. Process for the preparation of the cyclodextrin derivatives according to Claims 1 to 3, characterized in that an α-aminodicarboxylic acid, the α-amino acid functional group of which has been protected and which has the following formula: 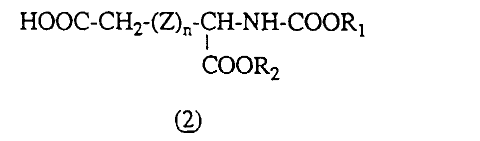 in which:
Z and n are as defined in Claim 1,
R₁ represents the tert-butyl radical or the benzyl radical, and
R₂ represents the methyl radical or the benzyl radical, is reacted in solution in an organic solvent with a mono(6-amino-6-deoxy)cyclodextrin of formula: CDNH₂
in which: CD is as defined in Claim 1, the reaction being carried out in the presence of 1-hydroxybenzotriazole and of dicyclohexylcarbodiimide, and in that the protected α-amino acid functional group is then deprotected.

5. Process according to Claim 4, characterized in that the organic solvent is dimethylformamide.

6. Process according to Claim 4 or 5, characterized in that the 1-hydroxybenzotriazole is used in a molar excess not exceeding 20% with respect to the protected α-aminodicarboxylic acid and the dicyclohexylcarbodiimide in a molar excess not exceeding 30% with respect to the protected α-aminodicarboxylic acid.

7. Process according to any one of Claims 4 to 6, characterized in that the molar ratio of the mono(6-amino-6-deoxy)cyclodextrin to the protected α-aminodicarboxylic acid is between 1 and 1.5.

8. Process according to any one of Claims 4 to 7, characterized in that the mono(6-amino-6-deoxy)cyclodextrin is mono(6-amino-6-deoxy)-β-cyclodextrin.

9. Process according to any one of Claims 4 to 8, characterized in that the deprotection is carried out by hydrogenation in the presence of Pd/C (5 to 10%) when R₁ and R₂ represent a benzyl radical in the formula (2).

10. Process according to any one of Claims 4 to 8, characterized in that deprotection is carried out in the presence of trifluoroacetic acid when R₁ represents a tert-butyl radical in the formula (2), deprotection of the ester functional group being obtained by saponification in the presence of sodium hydroxide.

11. Process according to any one of Claims 4 to 9, characterized in that the protected α-aminodicarboxylic acid is chosen from:
- benzyl N-(benzyloxycarbonyl)aspartate,
- benzyl N-(benzyloxycarbonyl)glutamate, and
- methyl N-(tert-butyloxycarbonyl)-O-(carboxymethyl)tyrosinate.

12. Intermediates in the synthesis of the cyclodextrin derivatives, characterized in that they correspond to the following general formula: 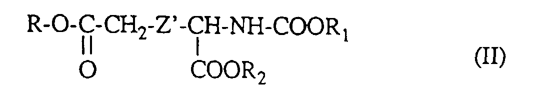 in which:
R represents a hydrogen atom or the benzyl radical,
R₁ represents the tert-butyl radical or the benzyl radical,
R₂ represents the methyl radical or the benzyl radical, and
Z' represents a divalent radical chosen from:
(i) -O-CH₂-
(ii) -S-CH₂- 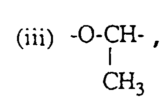
and
(iv) -O-C₆H₄-CH₂-.

13. Intermediates according to Claim 12, characterized in that they are chosen from: methyl N-(tert-butoxycarbonyl)-O-(benzyloxycarbonylmethyl)tyrosinate and methyl N-(tert-butoxycarbonyl)-O-(carboxymethyl)tyrosinate.

14. Process for the preparation of the intermediates according to Claims 12 and 13, characterized in that it consists in reacting benzyl bromoacetate with a protected ω-hydroxy- or ω-mercapto-α-amino acid of formula: 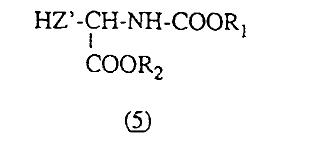 in which:
the HZ'-group represents the HO-CH₂-, HS-CH₂-, 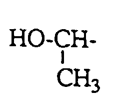 or p-OH-C₆H₄-CH₂- radical,
R₁ represents the tert-butyl radical or the benzyl radical, and
R₂ represents the methyl radical or the benzyl radical, in an organic solvent in the presence of lithium hydride and in optionally subjecting the product obtained of formula: 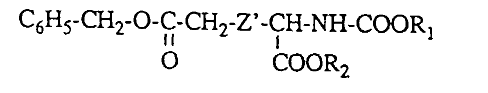 to a deprotection by hydrogenation in methanol/methylene chloride solution in the presence of Pd/C with a view to obtaining the corresponding acid.

15. Process according to Claim 14, characterized in that the protected ω-hydroxy-α-amino acid of formula (5) is methyl N-(tert-butyloxycarbonyl)tyrosinate.

16. Cosmetic or dermatological composition, characterized in that it contains from 0.1 to 10% by weight of at least one cyclodextrin derivative according to Claims 1 to 3 or obtained according to Claims 4 to 11.

17. Use of the cyclodextrin derivatives according to Claims 1 to 3 in the formation of inclusion complexes of active substances of lipophile nature or which are insoluble or unstable in aqueous medium.

18. Use according to Claim 17, characterized in that the active substances are chosen from anti-oxidizing agents, compounds which are active against free radicals, anti-acne agents, anti-aging agents, anti-psoriatic agents, preserving agents, bactericidal agents, agents promoting hair regrowth, agents for hydrating and/or plasticizing the stratum corneum, sunscreening agents, agents for reconstituting the lipid barrier, hair dyes, depigmenting agents, anti-inflammatory agents, anti-viral agents, anti-cancer agents and steroids.

19. Cosmetic or dermatological composition, characterized in that it contains an inclusion complex formed using a cyclodextrin derivative according to any one of Claims 1 to 3 and at least one active substance as defined according to Claim 18.

20. Cosmetic or dermatological composition according to Claim 19, characterized in that it contains the inclusion complex in a proportion of between 0.1 and 30% with respect to the total weight of the composition.
